# EUROPÄISCHE PATENTSCHRIFT

(11) **EP 1 133 263 B1**
(45) Veröffentlichungstag und Bekanntmachung des Hinweises auf die Patenterteilung: **20.07.2005**
(21) Anmeldenummer: 98954103.2
(22) Anmeldetag: 26.11.1998
(51) Int. Cl.: A61B 17/86, F16B 25/00

(54) **SCHRAUBE**
SCREW
VIS

(43) Veröffentlichungstag der Anmeldung: 19.09.2001
(73) Patentinhaber: SYNTHES AG Chur, 7002 Chur (CH)
(72) Erfinder: SCHLÄPFER, Fridolin, J., CH-8750 Glarus (CH); HESS, Martin, CH-4434 Hölstein (CH)
(74) Vertreter: Lusuardi, Werther Giovanni, Dr.
(86) Internationale Anmeldenummer: PCT/CH1998/000504
(87) Internationale Veröffentlichungsnummer: WO 2000/032125

(56) Entgegenhaltungen:
- EP-A- 0 491 211
- EP-A- 0 669 110
- WO-A-90/02526
- DE-C- 60 330
- US-A- 2 382 019
- US-A- 2 871 752

## Beschreibung

Die Erfindung betrifft eine knochenschraube gemäss dem Oberbegriff des Patentanspruchs 1.

Solche Schrauben finden insbesondere Verwendung in Stab- oder Plattensystemen für die dorsale Fixation von Instabilitäten der Wirbelsäule. Diese Systeme bestehen generell gesehen aus einem Längsträger und daran befestigbaren Verankerungselementen. Die Verankerungselemente sind entweder Haken oder durch die Pedikel geführte Schrauben, sogenannte Pedikelschrauben. Der Vorteil der Pedikelschrauben gegenüber den Haken ist deren intrinsische Stabilität im Knochen.

Grundsätzlich kann bei einer Pedikelschraube zwischen einem zur Verbindung mit dem Längsträger dienenden Oberteil (Kopf) und einem im Knochen zu verankemden Unterteil (Schaft) unterschieden werden. Die Ausbildung des im Knochen verankerten Unterteils spielt eine sehr wichtige Rolle bezüglich der Kurzzeit- und der Langzeit-Stabilität der Fixation. Die Kurzzeitstabilität ist rein mechanischer Natur, während die Langzeitstabilität stark durch das Zusammenspiel von Mechanik und Biologie am Übergang (interface) zwischen Knochen und Schraube und die Ermüdungsfestigkeit der Pedikelschraube selbst beeinflusst wird.

Pedikelschrauben werden hauptsächlich auf Biegung und Zug belastet. Zu grosse Biegebelastung kann zu einem "traumatischen" oder "biologischen" Durchschneiden der Pedikelschraube im Knochen und/oder Brechen der Pedikelschraube selbst führen. Das Brechen einer Pedikelschraube ist immer ein mechanisches Versagen durch Ermüdung. Zu grosse Zugbelastung gefährdet nur das Knochen/Schrauben-Interface aber nicht die Schraube selbst.

Prinzipiell sind Schrauben mit drei verschiedenen Ausführungsformen von Gewinden bekannt:

### 1. Schrauben mit zylindrischem Gewinde

Bei diesen Schrauben ist sowohl der Kern als auch das Gewinde kreiszylindrisch ausgebildet. Von allen Schraubentypen mit gleichem Aussendurchmesser weist diese Form die höchste Ausreissfestigkeit auf. Dazu kommt, dass beim Zurückdrehen der Schraube (Distanz < 10 % der Gesamtlänge) die Ausreissfestigkeit kaum beeinflusst wird. Diese Form hat aber gegenüber den anderen Schraubentypen folgende Nachteile:
- Schlechtes Fassen im Knochen, wenn nicht gut vorgebohrt wurde;
- Erweiterung der Eintrittsstelle durch "eierndes Eindrehen (bedingt durch den von den Weichteilen ausgeübten seitliche Druck) wird nicht kompensiert; und
- "Ausleiern" des Knochengewindes beim Eindrehen (je näher bei der Eintrittsstelle und je länger die Schraube, umso ausgeprägter ist das "Ausleiern").

### 2. Schrauben mit voll konischem Gewinde

Bei diesen Schrauben ist sowohl der Kern als auch das Gewinde konisch ausgebildet. Der Vorteil bei diesem Schraubentyp liegt im Fassen und Verspannen im Knochen. Nachteilig bei diesem Typus ist das Potential zum Durchschneiden im relativ weichen, spongiösen Knochen des Wrbelkörpers. Im weiteren darf die Schraube nicht mehr zurückgedreht werden, ohne eine Lockerung zu riskieren.

### 3. Schrauben mit teilkonischem Gewinde

Bei diesen Schrauben ist der Kern konisch und das Gewinde selbst kreiszylindrisch ausgebildet. Wie beim Schraubentyp mit zylindrischem Gewinde und Kern weist dieser Schraubentyp auf Grund des zylindrischen Gewindes eine hohe Ausreisskraft auf. Diese Schraube darf aber ebenfalls nicht zurückgedreht werden, ohne eine Lockerung zu riskieren. Das Potential zum Durchschneiden ist zwar geringer als beim voll konischen Gewinde, aber immer noch vorhanden. Je länger die Schraube ist, desto grösser ist das Potential den Knochen mit den sich gegen den Kopfteil hin immer mehr verdickenden Gewindespitzen zu sprengen. Die Verdickung der Gewindespitzen liegt in der Art des Gewindes begründet und kann dementsprechend nicht verhindert oder gesteuert werden. Je länger die Schraube, umso grösser ist die Verdickung der Gewindespitzen.

Aus der EP-A1 0 491 211 und US-A 2 382 019 sind Schrauben vom 1. Typus bekannt, welche zwei kreiszylindrische Schaftabschnitte mit unterschiedlichen Kern- und Aussendurchmesser aufweisen, welche durch einen konischen Übergangsteil miteinander verbunden sind. Der Vorderteil ist gewindelos ausgebildet. Nachteilig bei dieser bekannten Knochenschraube ist, dass die Schraube auf Grund des gewindelos ausgebildeten Vorderteils ohne gutes Vorbohren und grossen Axialdruck nur schlecht im Knochen fasst. Bei sprödem, sklerotischem Knochen kann es sogar geschehen, dass durch das schlechte Fassen der Schraubenspitze der Eindrehwiderstand so gross wird, dass das Knochengewinde beim Versuch, die Schraube durch die sklerotische Zone zu treiben, ausreisst. Aus der EP-A-0 669 110 und der US 2,382,019 sind zwar Schrauben bekannt, bei welchen auch der Vorderteil ein Gewinde trägt, doch fehlt es diesen Schrauben an einer abschnittsweisen, mehrstufigen Gestaltung des Schraubenkems.

Aus der US-A-2 871 752 STERN ist eine selbstbohrende Schraube bekannt, welche aber nicht speziell für chirurgische Zwecke vorgesehen ist. Sie weist einen im wesentlichen konstanten Kern auf, der in eine scharfe Spitze ausläuft, welche bei der vorgesehenen Anwendung in Holz oder Metall vorteilhaft sein kann, jedoch in der Chirurgie zur Sprengung des Knochens führen kann.

Eine Knochenschraube gemäß dem Oberbegriff von Anpruch 1 ist aus der EP-A-0 491 211 bekannt.

Der Erfindung liegt die Aufgabe zugrunde, eine Schraube zu schaffen; welche eine verbesserte Ausreiss-, Durchschneide- und Biegefestigkeit sowie auch ein gutes, von der Knochenqualität mehr oder weniger unabhängiges Fassen im Knochen aufweist.

Die Erfindung löst die gestellte Aufgabe mit einer Schraube, welche die Merkmale des Anspruchs 1 aufweist.

Durch den konstanten Aussendurchmesser des mit einem Gewinde versehenen Schaftes der Schraube ergibt sich eine optimale Ausreissfestigkeit, welche insbesondere bei einer Anwendung als Knochenschraube, im speziellen als Pedikelschraube, von besonderer klinischer Relevanz ist.

Der gewindete Vorderteil der Schraube, der sich gegen die Spitze hin verjüngt, weist gegenüber dem Stand der Technik folgende Vorteile auf:
- die Schraube fasst ohne zusätzlichen Axialdruck im Knochen;
- der gewindete, bombierte Vorderteil treibt die Schraube ohne zusätzlichen Axialdruck auch in spröden, sklerotischen Knochen vorwärts mit minimalem Risiko des Ausreissens des Knochengewindes auf Grund eines zu grossen Eindrehwiderstandes und mit minimalem Risiko einer Sprengung des Knochens;
- der gewindete, zugespitzte Vorderteil zentriert und führt die Schraube bei einer spinalen Anwendung im Pedikel und minimiert damit das Risiko einer seitlichen Penetration der Schraube im Pedikel.

Die Kombination des einen geringeren konstanten Kerndurchmesser aufweisenden Abschnitts mit dem, einen grösseren, ebenfalls konstanten Kerndurchmesser aufweisenden Abschnitt des Mittelteils der Schraube weist folgende Vorteile auf:
- das durch ein "eiemdes" Eindrehen der Schraube bewirkte Aufweiten der Eintrittsstelle wird durch den erweiterten Kerndurchmesser des einen grösseren, konstanten Kerndurchmesser aufweisenden Abschnittes kompensiert;
- die gute Verankerung im Bereich der Eintrittsstelle kombiniert mit einer guten Verankerung im Bereich der Schraubenspitze bietet eine optimale Durchschneidefestigkeit der Schraube im Knochen;
- dank der kreiszylindrischen Ausführung der beiden Abschnitte mit konstantem Kerndurchmesser ist die Ausreiss- und Durchschneidefestigkeit der Schraube im Knochen mehr oder weniger unabhängig von einem Zurückdrehen der Schraube innerhalb von ca. 10 % der Gesarntschraubenlänge;
- die Dicke der Gewindespitzen im einem grösseren, konstanten Kerndurchmesser aufweisenden Abschnitt ist über die gesamte Länge des Abschnitts konstant und hängt von der Differenz der Kerndurchmesser der beiden kreiszylindrischen Abschnitte ab;
- je grösser die Differenz, umso dicker sind die Gewindespitzen im kreiszylindrischen Abschnitt mit dem grösseren Kerndurchmesser; damit ist der Verkeileffekt der verdickten Gewindespitzen des kreiszylindrischen Abschnitts mit dem grösseren Kerndurchmesser im Knochen im Gegensatz zum 3. Schraubentyp mit einem teilkonischen Gewinde steuerbar.
- durch die Variation der Gesamtlänge der Schraube nur im kreiszylindrischen Abschnitts mit dem kleineren Kerndurchmesser kann die Länge des kreiszylindrischen Abschnitts mit dem grösseren Kerndurchmesser konstant gehalten werden. Damit kann der Verkeileffekt des kreiszylindrischen Abschnitts mit dem grösseren Kerndurchmesser im Knochen optimiert werden.

Der zwischen den beiden kreiszylindrischen Abschnitten liegende Abschnitt mit konischem Kern minimiert beim Übergang vom kleineren zum grösseren Kerndurchmesser die Sprengwirkung. Dieser Abschnitt bewirkt eine zusätzliche Verkeilung der Schraube im Knochen. Die Verkeilung wird bei jeglichem Zurückdrehen der Schraube drastisch reduziert. Die Länge des Abschnitts mit konischem Kern ist aber so kurz bemessen im Vergleich zur Länge der beiden kreiszylindrischen Abschnitte, dass der Einfluss dieses Abschnittes auf die Ausreiss- und Durchschneidefestigkeit der Schraube im Knochen verschwindend klein ist.

Eine bevorzugte Weiterbildung besteht darin, dass der sich gegen die Spitze hin verjüngende, bombierte Vorderteil mit einem parabelförmigen Profil ausgebildet ist. Die Vorteile liegen im guten Fassen des Gewindes, in der Zentrierung und Führung im Pedikel und in einem reduzierten Risiko einer Sprengung des Knochens.

Eine weitere bevorzugte Ausführungsform besteht darin, dass der Vorderteil an seiner Spitze eine in Richtung der zentralen Achse verlaufende, gewindelose Führungsnase aufweist. Die Führungsnase weist vorzugsweise den gleichen Durchmesser auf, wie die im Knochen vorzunehmende Bohrung und führt die Schraube entlang der Bohrung im Knochen, so dass die Gefahr einer seitlichen Penetrierung des Pedikels verringert wird. Vorzugsweise ist die Führungsnase kreiszylindrisch ausgebildet.

Eine weitere bevorzugte Ausführungsform besteht darin, dass das Gewinde als zweigängiges Gewinde ausgebildet ist. Damit ist der Vorteil erzielbar, dass bei gleicher Haltekraft im Knochengewebe, die Pedikelschraube pro Umdrehung eine x-fache Axialverschiebung entlang der Schraubenachse ausführt und somit schneller in den Knochen eingedreht werden kann, wie dies mit eingängigen Schrauben nach dem Stand der Technik möglich ist. Das zweigängige Gewinde weist zudem ein geringeres Potential zum Durchschneiden im Wirbelkörper auf, weil die Gewindeflanken auf Grund der grossen Steigung eine stützende Wirkung entfalten. Schliesslich ist auch die Zentrierung im Gewindeloch verbessert.

Die Erfindung und Weiterbildungen der Erfindung werden im folgenden anhand der teilweise schematischen Darstellungen eines Ausführungsbeispiels noch näher erläutert.

Es zeigen:
Fig. 1 einen schematischen Längsschnitt durch die erfindungsgemässe Schraube; und
Fig. 2 einen vergrösserten Teilausschnitt des Gewindes der Schraube nach Fig. 1 im Bereich des sich verjüngenden Abschnittes des Mittelteils.

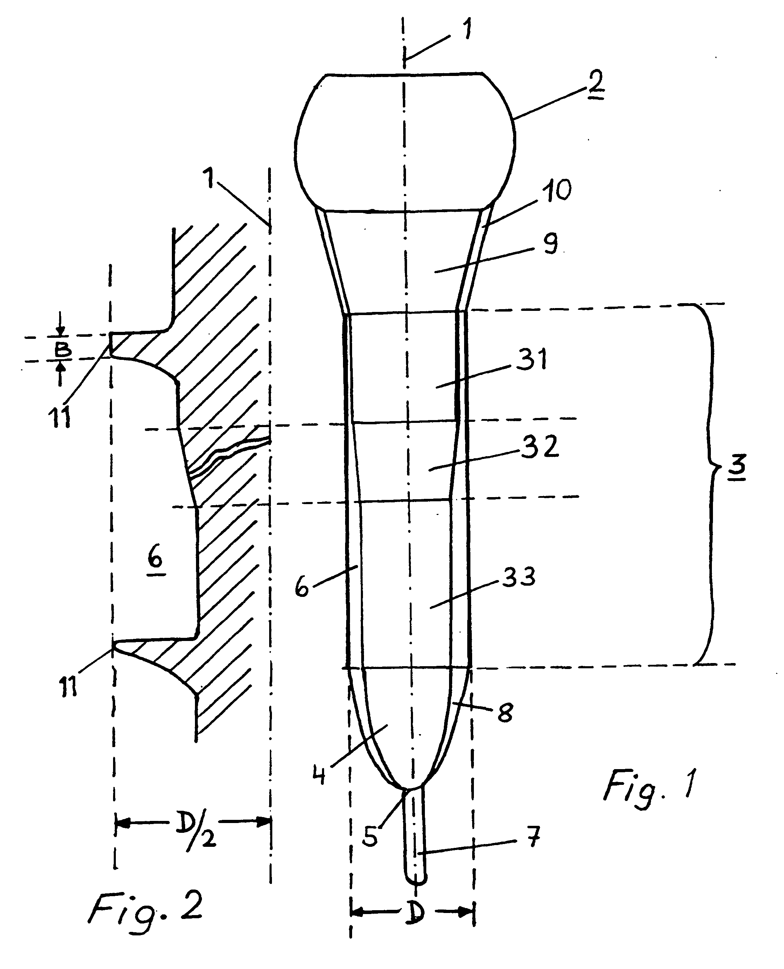

Die in den Fig. 1 und 2 dargestellte Schraube weist eine zentrale Achse 1, einen Kopfteil 2, einen mit einem Gewinde 6 versehenen Mittelteil 3 und einem Vorderteil 4 mit einer Spitze 5 auf. Der Mittelteil 3 weist drei verschiedene Abschnitte 31, 32 und 33 auf, wobei der Kern des Abschnitts 33 kreiszylindrisch ausgebildet ist und an den Vorderteil 4 anschliesst, der an den Kopfteil 2 anschliessende Kern des Abschnitts 31 kreiszylindrisch ausgebildet ist und einen grösseren Durchmesser als der Kern des Abschnitts 33 aufweist und der Kern des Abschnitts 32 zwischen den beiden Abschnitten 31 und 33 liegt und sich in Richtung der Spitze 5 hin verjüngt. Die Abschnitte 31, 32 und 33 des Mittelteils 3 sind durchgehend mit dem Gewinde 6 versehen und der das durchgehende Gewinde 6 tragende Mittelteil 3 weist einen im wesentlichen konstanten Aussendurchmesser D auf.
Der Vorderteil 4 verjüngt sich gegen seine Spitze 5 hin und ist mit einem Gewinde 8 versehen. Die Spitze 5 weist eine in Richtung der zentralen Achse 1 verlaufende, gewindelose Führungsnase 7 mit kreiszylindrischer Form auf.

Der Kopfteil 2 ist teilkugelförmig ausgebildet, um die Schraube polyaxial einsetzen zu können. Der Kopfteil kann übliche Vertiefungen, z.B. hexagonaler Form ausweisen um ein entsprechendes Eindrehwerkzeug aufzunehmen.

Der Durchmesser der Schraube im Übergangsbereich 9 vom Mittelteil 3 zum Kopfteil 2 nimmt in Richtung des Kopfteils 2 zu, kann aber auch kreiszylindrisch ausgebildet sein. Im Übergangsbereich 9 vom Mittelteil 3 zum Kopfteil 2 ist ein Gewinde 10 vorgesehen. Der Kern- und Aussendurchmesser des Gewindes 10 im Übergangsbereich 9 verbreitert sich vom Mittelteil 3 zum Kopfteil 2 hin konisch. Das Gewinde 10 im Übergangsbereich 9 vom Mittelteil 3 zum Kopfteil 2 und das Gewinde 6 des Mittelteils 3 sind zusammenhängend und weisen die gleiche Steigung auf.

Das Gewinde 6 kann als zweigängiges Gewinde ausgebildet werden mit einem Steigungswinkel zwischen 10° und 22°, vorzugsweise zwischen 14° und 18°. Die Gewindesteigung beträgt 3 bis 5 mm, vorzugsweise 3,5 bis 4,5 mm. Die Gewindesteigung und Anzahl der Gewindegänge definieren die optimale Grösse der Gewindezwischenräume, welche eine mechanisch geschützte Ruhezone für Knochenzellen ergeben, was zu einer verbesserten biologischen Verankerung führt.

Das Gewinde 6 kann aber auch eingängig ausgebildet sein. In diesem Fall liegt der Steigungswinkel des Gewindes 6 zwischen 5° und 18°, vorzugsweise zwischen 8° und 14° und die Gewindesteigung liegt zwischen 1,5 mm und 4,0 mm, vorzugsweise zwischen 1,75 mm und 3,50 mm.

Die Tiefe des Gewindes 6 ist im Bereich des Abschnitts 31 grösser als 0,2 mm und beträgt typischerweise 0,4 mm bis 0,6 mm, vorzugsweise 0,5 mm. Bei einer alternativen Ausführung ist die Tiefe des Gewindes 6 im Bereich des Abschnitts 31 grösser als 0,5 mm und beträgt typischerweise 0,6 bis 1,0 mm, vorzugsweise 0,85 mm.

Das Gewinde 6 des Mittelteils 3 setzt sich im Vorderteil 4 fort. Der Vorderteil 4 verjüngt sich gegen die Spitze 5 hin konvex. Die konvexe Verjüngung läuft tangential in den Kern des Mittelteils 3 und weist im Profil einen Radius zwischen 30 und 50 mm, vorzugsweise zwischen 38 und 42 mm auf. Alternativ kann die konvexe Verjüngung tangential in den Kern des Mittelteils 3 laufen und im Profil parabelförmig ausgebildet sein.

Das Gewinde 8 des Vorderteils 4 und das Gewinde 6 des Mittel teils 3 weisen die gleiche Steigung und die gleiche Anzahl Gewindegänge auf. Das Gewinde 6 des Mittelteils 3 geht kontinuierlich in das Gewinde 8 des Vorderteils 4 über, das sich gegen die Spitze 5 hin konvex verjüngt. Die Verjüngung des Gewindes 8 des Vorderteils 4 weist im Profil einen Radius zwischen 20 mm und 40 mm auf. Es kann auch parabelförmig ausgebildet sein.

Der Vorderteil 4 endet zweckmässigerweise in einem Kugelradius zwischen 0,5 und 3,0 mm, vorzugsweise zwischen 1,5 und 2,0 mm.

Der Kern des Abschnitts 32 verjüngt sich konisch mit einem halben Konuswinkel im Bereich von 3° bis 7°. Der Kern des Abschnitts 31 ist 15 - 35 % grösser als der Kern des Abschnitts 33.

Wie in Fig. 2 im Detail dargestellt sind die Spitzen 11 des Gewindes 6 im Bereich des Abschnitts 31 dicker als im Bereich des Abschnitts 33. Die Differenz zwischen dem Kerndurchmesser des Abschnitts 33 und des Abschnitts 31 ist derart ausgelegt, dass die Spitzen 11 des Gewindes 6 im Bereich des Abschnitts 31 eine Breite B zwischen 0,3 und 0,5 mm aufweisen.

Der Abschnitt 33 kann seinerseits drei weitere (zeichnerisch nicht dargestellte) Teilabschnitte umfassen, welche in ihrer Form und Funktion in analoger Weise den drei Abschnitten 31, 32 und 33 des Mittelteils 3 entsprechen.

Nachstehend wird die Funktionsweise der erfindungsgemässen Schraube in ihrer Ausgestaltung als Pedikelschraube beschrieben.

Der Pedikel wird mit einem geeigneten Werkzeug, z.B. einer Ahle oder einem Bohrer eröffnet. Der Durchmesser der Bohrung soll dabei kleiner sein als der kleinste Kerndurchmesser der Schraube, d.h. des Abschnitts 33. Beim Eindrehen der Schraube erweitert der bombiert ausgestaltete Vorderteil 4 das Bohrloch im spongiösen Knochen mit minimaler Sprengwirkung auf den Kerndurchmesser des Abschnitts 33. Der verdrängte spongiöse Knochen wird im Bereich des Gewindes 6 komprimiert.

Oft wird das Eindrehen der Schraube durch die Weichteile behindert. Der seitliche Druck der Weichteile kann zu Beginn zu einem "eiernden" Eindrehen der Schraube führen und ein Aufweiten des Gewindeloches im Bereich der Eintrittsstelle bewirken. Mit zunehmender Eindringtiefe stabilisiert sich die Schraube aber immer mehr. Die Abfolge der beiden kreiszylindrischen Abschnitte 33 und 31 ist nun derart aufeinander abgestimmt, dass der einen grösseren Kerndurchmesser aufweisende Abschnitt 31 erst dann in den Knochen eindringt, wenn die Schraube vollends stabilisiert ist. Der einen konischen Kern aufweisende Abschnitt 32 erweitert dabei den Kerndurchmesser des Gewindeloches auf den Kerndurchmesser des Abschnitts 31. Mit zunehmendem Kerndurchmesser nimmt auch die Dicke der Gewindespitze 11 gleichmässig zu bis im Bereich des Abschnitts 31, wo sie wieder konstant ist. Der Abschnitt 31 ist so lang gewählt, dass er bis in den Pedikel reicht und sich in der verdichteten Randzone des Pedikels optimal verkrallen kann. Die Länge des Abschnitts 31 beträgt zwischen 10 und 30 mm, vorzugsweise 15 mm für Schrauben bis zur Gewindelänge von 45 mm und 20 mm für Schrauben mit Gewindelänge grösser als 45 mm. Die Dicke der Gewindespitze im Bereich des Abschnitts 31 hängt von der Differenz zwischen den beiden Kemdurchmessern der Abschnitte 33 und 31 ab. Um das Risiko eines Sprengens des Pedikels zu minimieren sind die beiden Kerndurchmesser derart aufeinander abgestimmt, dass die Dicke der Gewindespitze zwischen 0,2 und 0,5 mm, vorzugsweise zwischen 0,3 und 0,4 mm beträgt. Der eigentliche Längenausgleich der Schraube findet nur im Abschnitt 33 statt.

## Patentansprüche

1. Knochenschraube mit einer zentralen Achse (1), einem Kopfteil (2), einem mit einem Gewinde versehenen Mittelteil (3) und einem Vorderteil (4) mit einer Spitze (5), wobei der Mittelteil (3) in M Abschnitte (32), mit in Richtung des Kopfteils (2) sich erweiterndem Kern und N Abschnitte (31,33) mit kreiszylindrischem Kern aufgeteilt ist, wobei
(i) M und N ganzzahlig und grösser als O sind und maximal um den Betrag 1 differieren;
(ii) die Abschnitte (32), mit in Richtung des Kopfteils (2) sich erweiterndem Kern und die Abschnitte (31,33) mit kreiszylindrischem Kern alternierend abwechseln,
(iii) alle Abschnitte (31,32,33) des Mittelteils (3) durchgehend mit einem Gewinde (6) versehen sind;
(iv) der das durchgehende Gewinde (6) tragende Mittelteil (3) einen im wesentlichen konstanten Aussendurchmesser D aufweist,
(v) M = 1 und N = 2 ist, und
(vi) der sich gegen die Spitze hin verjüngende Vorderteil (4) bombiert ausgebildet ist,
**dadurch gekennzeichnet, dass**
(vii) der Vorderteil (4) sich gegen die Spitze (5) hin konvex verjüngt und ein Gewinde (8) aufweist.

2. Schraube nach Anspruch 1, **dadurch gekennzeichnet, dass** der Vorderteil (4) an seiner Spitze (5) eine in Richtung der zentralen Achse (1) verlaufende, gewindelose Führungsnase (7) aufweist.

3. Schraube nach Anspruch 1 oder 2, **dadurch gekennzeichnet, dass** der Kopfteil (2) teilkugelförmig ausgebildet ist.

4. Schraube nach einem der Ansprüche 1 bis 3, **dadurch gekennzeichnet, dass** der Übergang vom Mittelteil (3) zum Kopfteil (2) kreiszylindrisch ist.

5. Schraube nach einem der Ansprüche 1 bis 3, **dadurch gekennzeichnet, dass** ihr Durchmesser im Übergangsbereich (9) vom Mittelteil (3) zum Kopfteil (2) in Richtung des Kopfteils (2) zunimmt.

6. Schraube nach einem der Ansprüche 1 bis 5, **dadurch gekennzeichnet, dass** sie im Übergangsbereich (9) vom Mittelteil (3) zum Kopfteil (2) ein Gewinde (10) aufweist.

7. Schraube nach Anspruch 6, **dadurch gekennzeichnet, dass** der Kern- und Aussendurchmesser des Gewindes (10) im Übergangsbereich (9) vom Mittelteil (3) zum Kopfteil (2) hin sich konisch verbreitert.

8. Schraube nach Anspruch 6 oder 7, **dadurch gekennzeichnet, dass** das Gewinde (10) im Übergangsbereich (9) vom Mittelteil (3) zum Kopfteil (2) und das Gewinde (6) des Mittelteils (3) die gleiche Steigung aufweisen.

9. Schraube nach einem der Ansprüche 6 bis 8, **dadurch gekennzeichnet, dass** das Gewinde (10) im Übergangsbereich (9) vom Mittelteil (3) zum Kopfteil (2) und das Gewinde (6) des Mittelteils (3) zusammenhängend sind.

10. Schraube nach einem der Ansprüche 1 bis 9, **dadurch gekennzeichnet, dass** das Gewinde (6) als zweigängiges Gewinde ausgebildet ist.

11. Schraube nach einem der Ansprüche 1 bis 10, **dadurch gekennzeichnet, dass** der Steigungswinkel des Gewindes (6) zwischen 10° und 22° beträgt.

12. Schraube nach Anspruch 11, **dadurch gekennzeichnet, dass** der Steigungswinkel des Gewindes (6) zwischen 14° und 18° beträgt.

13. Schraube nach einem der Ansprüche 1 bis 12, **dadurch gekennzeichnet, dass** die Gewindesteigung zwischen 3 und 5 mm beträgt.

14. Schraube nach Anspruch 13, **dadurch gekennzeichnet, dass** die Gewindesteigung zwischen 3,5 und 4,5 mm beträgt.

15. Schraube nach einem der Ansprüche 1 bis 14, **dadurch gekennzeichnet, dass** das Gewinde (6) eingängig ist.

16. Schraube nach Anspruch 15, **dadurch gekennzeichnet, dass** der Steigungswinkel des Gewindes (6) zwischen 5° und 18° liegt.

17. Schraube nach Anspruch 16, **dadurch gekennzeichnet, dass** der Steigungswinkel des Gewindes (6) zwischen 8° und 14° liegt.

18. Schraube nach einem der Ansprüche 15 bis 17, **dadurch gekennzeichnet, dass** die Gewindesteigung des Gewindes (6) zwischen 1,5 mm und 4,0 mm liegt.

19. Schraube nach Anspruch 18, **dadurch gekennzeichnet, dass** die Gewindesteigung des Gewindes (6) zwischen 1,75 mm und 3,50 mm liegt.

20. Schraube nach einem der Ansprüche 1 bis 19, **dadurch gekennzeichnet, dass** das Gewinde (6) des Mittelteils (3) sich im Vorderteil (4) fortsetzt.

21. Schraube nach einem der Ansprüche 1 bis 20, **dadurch gekennzeichnet, dass** die konvexe Verjüngung tangential in den Kern des Mittelteils (3) läuft und im Profil einen Radius zwischen 30 und 50 mm, aufweist.

22. Schraube nach Anspruch 21, **dadurch gekennzeichnet, dass** die konvexe Verjüngung tangential in den Kern des Mittelteils (3) läuft und im Profil einen Radius zwischen 38 und 42 mm aufweist.

23. Schraube nach einem der Ansprüche 1 bis 22, **dadurch gekennzeichnet, dass** die konvexe Verjüngung tangential in den Kern des Mittelteils (3) läuft und im Profil parabelförmig ist.

24. Schraube nach einem der Ansprüche 1 bis 23, **dadurch gekennzeichnet, dass** das Gewinde (8) des Vorderteils (4) und das Gewinde (6) des Mittelteils (3) die gleiche Steigung und die gleiche Anzahl Gewindegänge aufweisen.

25. Schraube nach einem der Ansprüche 1 bis 24, **dadurch gekennzeichnet, dass** das Gewinde (6) des Mittelteils (3) kontinuierlich in das Gewinde (8) des Vorderteils (4) übergeht.

26. Schraube nach einem der Ansprüche 1 bis 25, **dadurch gekennzeichnet, dass** das Gewinde (8) des Vorderteils (4) kreiszylindrisch ausgebildet ist.

27. Schraube nach einem der Ansprüche 1 bis 26, **dadurch gekennzeichnet, dass** das Gewinde (8) des Vorderteils (4) sich gegen die Spitze (5) hin konvex verjüngt.

28. Schraube nach einem der Ansprüche 1 bis 27, **dadurch gekennzeichnet, dass** das Gewinde (8) des Vorderteils (4) sich gegen die Spitze (5) hin konisch verjüngt.

29. Schraube nach Anspruch 27 oder 28, **dadurch gekennzeichnet, dass** die Verjüngung des Gewindes (8) des Vorderteils (4) im Profil einen Radius zwischen 20 mm und 40 mm aufweist.

30. Schraube nach Anspruch 27 oder 29, **dadurch gekennzeichnet, dass** die Verjüngung des Gewindes (8) des Vorderteils (4) im Profil parabelförmig ist.

31. Schraube nach einem der Ansprüche 1 bis 30, **dadurch gekennzeichnet, dass** der Vorderteil (4) in einem Kugelradius zwischen 0,5 und 3,0 mm endet.

32. Schraube nach Anspruch 31, **dadurch gekennzeichnet, dass** der Vorderteil (4) in einem Kugelradius zwischen 1,5 und 2,0 mm endet.

33. Schraube nach einem der Ansprüche 1 bis 32, **dadurch gekennzeichnet, dass** der Kern des Abschnitts (32) sich konisch verjüngt mit einem halben Konuswinkel im Bereich von 3° bis 7°.

34. Schraube nach einem der Ansprüche 1 bis 35, **dadurch gekennzeichnet, dass** der Kern des Abschnitts (31) 15 - 35 % grösser ist als der Kern des Abschnitts (33).

35. Schraube nach einem der Ansprüche 1 bis 34, **dadurch gekennzeichnet, dass** die Spitzen (11) des Gewindes (6) im Bereich des Abschnitts (31) dicker sind als im Bereich des Abschnitts (33).

36. Schraube nach einem der Ansprüche 1 bis 35, **dadurch gekennzeichnet, dass** die Differenz zwischen dem Kerndurchmesser des Abschnitts (33) und des Abschnitts (31) derart ausgelegt ist, dass die Spitzen (11) des Gewindes (6) im Bereich des Abschnitts 31 eine Breite B zwischen 0,3 und 0,5 mm aufweisen.

37. Schraube nach einem der Ansprüche 1 bis 36, **dadurch gekennzeichnet, dass** der Abschnitt (33) seinerseits mindestens drei Teilabschnitte umfasst, welche in ihrer Form und Funktion in analoger Weise den drei Abschnitten (31,32,33) des Mittelteils (3) entsprechen.

38. Schraube nach einem der Ansprüche 1 bis 37, **dadurch gekennzeichnet, dass** die Tiefe des Gewindes (6) im Bereich des Abschnitts (31) grösser als 0,2 mm ist.

39. Schraube nach Anspruch 38, **dadurch gekennzeichnet, dass** die Tiefe des Gewindes (6) im Bereich des Abschnitts (31) zwischen 0,4 mm und 0,6 mm liegt.

40. Schraube nach Anspruch 39, **dadurch gekennzeichnet, dass** die Tiefe des Gewindes (6) im Bereich des Abschnitts (31) grösser als 0,5 mm ist.

41. Schraube nach Anspruch 40, **dadurch gekennzeichnet, dass** die Tiefe des Gewindes (6) im Bereich des Abschnitts (31) zwischen 0,6 und 1,0 mm liegt.

## Claims

1. A bone screw having a central axis (1), a head part (2), a central part (3) fitted with a thread, and a front part (4) comprising a tip (5), where the central part (3) is divided into M segments (32) having a body widening in the direction of the head part (2) and into N segments (31, 33) of circular-cylindrical cross-section, where
a) M and N are integral numbers larger than 0 and maximally differing by the magnitude 1; and
b) The segments (32) having a body widening in the direction of the head part (2) and the segments (31, 33) having a circular-cylindrical body mutually alternate,
c) All segments (31, 32, 33) of the central part (3) are fitted with a continuous thread (6),
d) The central part ((3) supporting the continuous thread (6) comprises a substantially constant outside diameter D,
e) M =1 and N = 2, and
f) the front part (4) tapers in convex manner toward the tip (5),
**characterized in that**
g) the front part (4) convexedly tapers toward the tip (5) and comprises a thread (8).

2. Screw as claimed in claim 1, **characterized in that** the front part (4) is fitted at its tip (5) with an unthreaded guide beak (7) running in the direction of the central axis (1).

3. Screw as claimed in claim 1 or 2, **characterized in that** the head part (2) is frusto-spherical.

4. Screw as claimed in one of claims 1 through 3, **characterized in that** the transition structure from the central part (3) to the head part (2) is circular-cylindrical.

5. Screw as claimed in one of claims 1 through 3, **characterized in that** its diameter increases in the transition range (9) from the central part (3) to the head part (2) toward this head part (2).

6. Screw as claimed in one of claims 1 through 5, **characterized in that** it comprises a thread (10) in the transition range (9) from the central part (3) to the head part (2).

7. Screw as claimed in claim 6, **characterized in that** the inside and outside diameters of the thread (10) conically widen in the transition range (9) from the central part (3) toward the head part (2).

8. Screw as claimed in either of claims 6 and 7, **characterized in that** the thread (10) in the transition range (9) from the central part (3) to the head part (2) and the thread (6) of the central part (3) are of the same pitch.

9. Screw as claimed in one of claims 6 through 8, **characterized in that** the thread (10) in the transition range (9) from the central part (3) to the head part (2) and the thread (6) of the central part (3) match turn to turn.

10. Screw as claimed in one of claims 1 through 9, **characterized in that** the thread (6) is a double-thread.

11. Screw as claimed in one of claims 1 through 10, **characterized in that** the thread angle of the thread (6) is between 10 and 22°.

12. Screw as claimed in claim 11, **characterized in that** the thread angle of the thread (6) is between 14 and 18°.

13. Screw as claimed in one of claims 1 through 12, **characterized in that** the thread pitch is between 3 and 5 mm.

14. Screw as claimed in claim 13, **characterized in that** the thread pitch is between 3.5 and 4.5 mm.

15. Screw as claimed in one of claims 1 through 14, **characterized in that** the thread (6) is a single thread.

16. Screw as claimed in claim 15, **characterized in that** the thread angle of the thread (6) is between 5 and 18°.

17. Screw as claimed in claim 16, **characterized in that** the thread angle of the thread (6) is between 8 and 14°.

18. Screw as claimed in one of claims 15 through 17, **characterized in that** the pitch of the thread (6) is between 1.5 and 4.0 mm.

19. Screw as claimed in claim 18, **characterized in that** the pitch of the thread (6) is between 1.75 and 3.50 mm.

20. Screw as claimed in one of claims 1 through 19, **characterized in that** the thread (6) of the central part (3) continues into the front part (4).

21. Screw as claimed in one of the claims 1 through 20, **characterized in that** the convex taper runs tangentially into the body of the central part (3) and has a cross-sectional radius between 30 and 50 mm.

22. Screw as claimed in claim 21, **characterized in that** the convex taper runs tangentially into the body of the central part (3) and has a cross-sectional radius between 38 and 42 mm.

23. Screw as claimed in one of the claims 1 through 22, **characterized in that** the convex taper runs tangentially into the body of the central part (3) and is cross-sectionally parabolic.

24. Screw as claimed in one of claims 1 through 23, **characterized in that** the thread (8) of the front part (4) and the thread (6) of the central part (3) comprise the same pitch and the same number of thread turns.

25. Screw as claimed in one of claims 1 through 24, **characterized in that** the thread (6) of the central part (3) continuously merges into the thread (8) of the front part (4).

26. Screw as claimed in one of claims 1 through 25, **characterized in that** the thread (8) of the front part (4) is circular-cylindrical.

27. Screw as claimed in one of claims 1 through 26, **characterized in that** the thread (8) of the front part (4) tapers in convex manner toward the tip (5).

28. Screw as claimed in one of claims 1 through 27, **characterized in that** the thread (8) of the front part (4) tapers conically toward the tip (5).

29. Screw as claimed in either of claims 27 and 28, **characterized in that** the taper of the thread (8) of the front part (4) cross-sectionally has a radius between 20 and 40 mm.

30. Screw as claimed in either of claims 27 and 29, **characterized in that** the taper of the thread (8) of the front part (4) is cross-sectionally parabolic.

31. Screw as claimed in one of claims 1 through 30, **characterized in that** the front part (4) terminates at a spherical radius between 0.5 and 3.0 mm.

32. Screw as claimed in claim 31, **characterized in that** the front part (4) terminates in a spherical radius between 1.5 and 2.0 mm.

33. Screw as claimed in one of claims 1 through 32, **characterized in that** the body of the segment (32) tapers conically at a half-conical angle in the range of 3 to 7°.

34. Screw as claimed in one of claims 1 through 33, **characterized in that** the body of the segment (31) is larger by 15 to 35 % than the body of the segment (33).

35. Screw as claimed in one of claims 1 through 34, **characterized in that** the tops (11) of the thread (6) are thicker in the region of the segment (31) than in the region of the segment (33).

36. Screw as claimed in one of claims 1 through 35, **characterized in that** the differential of the body diameter of the segment (33) and of the segment (31) is selected in such manner that the tops (11) of the thread (6) are a width B between 0.3 and 0.5 mm in the region of the segment (31).

37. Screw as claimed in one of claims 1 through 36, **characterized in that** the segment (33) itself comprises at least three sub-segments of which the shape and function correspond similarly to the three segments (31, 32, 33) of the central part (3).

38. Screw as claimed in one of claims 1 through 37, **characterized in that** the depth of the thread (6) is larger than 0.2 mm in the region of the segment (31).

39. Screw as claimed in claim 38, **characterized in that** the depth of the thread (6) is between 0.4 and 0.6 mm in the region of the segment (31).

40. Screw as claimed in claim 39, **characterized in that** the depth of the thread (6) is larger than 0.5 mm in the region of the segment (31).

41. Screw as claimed in claim 40, **characterized in that** the depth of the thread (6) is between 0.6 and 1.0 mm in the region of the segment (31).

## Revendications

1. Vis d'ostéosynthèse comportant un axe central (1), une tête (2), une partie centrale (3) pourvue d'un filetage et une partie avant (4) avec une pointe (5), la partie centrale (3) étant subdivisée en M zones (32) comportant un noyau allant en s'élargissant vers la tête (2) et N zones (31,33) comportant un noyau cylindrique circulaire,
(i) M et N étant des nombres entiers et supérieurs à 0 et leur différence étant au maximum égale à la valeur 1;
(ii) les zones (32) comportant un noyau allant en s'élargissant vers la tête (2) et les zones (31,33) comportant un noyau cylindrique circulaire étant disposées en alternance les unes avec les autres;
(iii) toutes les zones (31,32,33) constituant la partie centrale (3) étant pourvues en continu d'un filetage (6);
(iv) la partie centrale (3) pourvue du filetage continu (6) présentant un diamètre extérieur D essentiellement constant;
(v) M = 1 et N = 2, et
(vi) la partie avant (4) allant en s'amincissant vers la pointe étant de forme bombée,
**caractérisée en ce que**
(vii) la partie avant (4) va en s'amincissant de manière convexe vers la pointe (5) et présente un filetage (8).

2. Vis selon la revendication 1, **caractérisée en ce que** la partie avant (4) présente, au niveau de sa pointe (5), un bec de guidage (7) dépourvu de filetage et s'étendant le long de l'axe central (1).

3. Vis selon la revendication 1 ou 2, **caractérisée en ce que** la tête (2) est réalisée en forme de partie sphérique.

4. Vis selon l'une des revendications 1 à 3, **caractérisée en ce que** la transition entre la partie centrale (3) et la tête (2) est de forme cylindrique circulaire.

5. Vis selon l'une des revendications 1 à 3, **caractérisée en ce que** son diamètre au niveau de la partie de transition (9) entre la partie centrale (3) et la tête (2) va en augmentant vers la tête (2).

6. Vis selon l'une des revendications 1 à 5, **caractérisée en ce que** la partie de transition (9) entre la partie centrale (3) et la tête (2) présente un filetage (10).

7. Vis selon la revendication 6, **caractérisée en ce que** le diamètre de noyau et le diamètre extérieur du filetage (10) au niveau de la zone de transition (9) vont en s'élargissant de manière conique depuis la partie centrale (3) vers la tête (2).

8. Vis selon la revendication 6 ou 7, **caractérisée en ce que** le filetage (10) au niveau de la partie de transition (9) entre la partie centrale (3) et la tête (2) ainsi que le filetage (6) de la partie centrale (3) présentent le même pas.

9. Vis selon l'une des revendications 6 à 8, **caractérisée en ce que** le filetage (10) au niveau de la partie de transition (9) entre la partie centrale (3) et la tête (2) ainsi que le filetage (6) de la partie centrale (3) sont d'un même tenant.

10. Vis selon l'une des revendications 1 à 9, **caractérisée en ce que** le filetage (6) est un filetage double.

11. Vis selon l'une des revendications 1 à 10, **caractérisée en ce que** l'angle de pas du filetage (6) est compris entre 10° et 22°.

12. Vis selon la revendication 11, **caractérisée en ce que** l'angle de pas du filetage (6) est compris entre 14° et 18°.

13. Vis selon l'une des revendications 1 à 12, **caractérisée en ce que** le pas de filetage est compris entre 3 et 5 mm.

14. Vis selon la revendication 13, **caractérisée en ce que** le pas de filetage est compris entre 3,5 et 4,5 mm.

15. Vis selon l'une des revendications 1 à 14, **caractérisée en ce que** le filetage (6) est un filetage simple.

16. Vis selon la revendication 15, **caractérisée en ce que** l'angle de pas du filetage (6) est compris entre 5° et 18°.

17. Vis selon la revendication 16, **caractérisée en ce que** l'angle de pas du filetage (6) est compris entre 8° et 14°.

18. Vis selon l'une des revendications 15 à 17, **caractérisée en ce que** l'angle de pas du filetage (6) est compris entre 1,5 mm et 4,0 mm.

19. Vis selon la revendication 18, **caractérisée en ce que** l'angle de pas du filetage (6) est compris entre 1,75 mm et 3,50 mm.

20. Vis selon l'une des revendications 1 à 19, **caractérisée en ce que** le filetage (6) de la partie centrale (3) se poursuit dans la partie avant (4).

21. Vis selon l'une des revendications 1 à 20, **caractérisée en ce que** la conicité convexe s'étend vers le noyau de la partie centrale (3) jusqu'à se confondre tangentiellement avec celui-ci et présente, en coupe transversale, un rayon compris entre 30 et 50 mm.

22. Vis selon la revendication 21, **caractérisée en ce que** la conicité convexe s'étend vers le noyau de la partie centrale (3) jusqu'à se confondre tangentiellement avec celui-ci et présente, en coupe transversale, un rayon compris entre 38 et 42 mm.

23. Vis selon l'une des revendications 1 à 22, **caractérisée en ce que** la conicité convexe s'étend vers le noyau de la partie centrale (3) jusqu'à se confondre tangentiellement avec celui-ci et présente, en coupe transversale, une forme de parabole.

24. Vis selon l'une des revendications 1 à 23, **caractérisée en ce que** le filetage (8) de la partie avant (4) et le filetage (6) de la partie centrale (3) présentent le même pas et le même nombre de spires.

25. Vis selon l'une des revendications 1 à 24, **caractérisée en ce que** le filetage (6) de la partie centrale (3) se poursuit de manière continue dans le filetage (8) de la partie avant (4).

26. Vis selon l'une des revendications 1 à 25, **caractérisée en ce que** le filetage (8) de la partie avant (4) est de forme cylindrique circulaire.

27. Vis selon l'une des revendications 1 à 26, **caractérisée en ce que** le filetage (8) de la partie avant (4) va en s'amincissant de manière convexe vers la pointe (5).

28. Vis selon l'une des revendications 1 à 27, **caractérisée en ce que** le filetage (8) de la partie avant (4) va en s'amincissant de manière conique vers la pointe (5).

29. Vis selon la revendication 27 ou 28, **caractérisée en ce que** la conicité du filetage (8) de la partie avant (4) présente, en coupe transversale, un rayon compris entre 20 mm et 40 mm.

30. Vis selon la revendication 27 ou 29, **caractérisée en ce que** la conicité du filetage (8) de la partie avant (4) présente, en coupe transversale, une forme de parabole.

31. Vis selon l'une des revendications 1 à 30, **caractérisée en ce que** la partie avant (4) se termine par un segment sphérique d'un rayon compris entre 0,5 et 3,0 mm.

32. Vis selon la revendication 31, **caractérisée en ce que** la partie avant (4) se termine par un segment sphérique d'un rayon compris entre 1,5 et 2,0 mm.

33. Vis selon l'une des revendications 1 à 32, **caractérisée en ce que** le noyau de la zone (32) va en s'amincissant de manière conique avec un demi-angle de cône compris entre 3° et 7°.

34. Vis selon l'une des revendications 1 à 35, **caractérisée en ce que** le noyau de la zone (31) est 15 à 35 % supérieur au noyau de la zone (33).

35. Vis selon l'une des revendications 1 à 34, **caractérisée en ce que** les crêtes (11) du filetage (6) sont plus épaisses au niveau de la zone (31) qu'au niveau de la zone (33).

36. Vis selon l'une des revendications 1 à 35, **caractérisée en ce que** la différence entre le diamètre de noyau de la zone (33) et celui de la zone (31) est conçue de telle sorte que les crêtes (11) du filetage (6) présentent, au niveau de la zone 31, une largeur B comprise entre 0,3 et 0,5 mm.

37. Vis selon l'une des revendications 1 à 36, **caractérisée en ce que** la zone (33) comprend, à son tour, au moins trois sections dont la forme et la fonction correspondent, de manière analogue, aux trois zones (31,32,33) constituant la partie centrale (3).

38. Vis selon l'une des revendications 1 à 37, **caractérisée en ce que** la profondeur du filetage (6) est, au niveau de la zone (31), supérieure à 0,2 mm.

39. Vis selon la revendication 38, **caractérisée en ce que** la profondeur du filetage (6) est comprise, au niveau de la zone (31), entre 0,4 mm et 0,6 mm.

40. Vis selon la revendication 39, **caractérisée en ce que** la profondeur du filetage (6) est, au niveau de la zone (31), supérieure à 0,5 mm.

41. Vis selon la revendication 40, **caractérisée en ce que** la profondeur du filetage (6) est comprise, au niveau de la zone (31), entre 0,6 mm et 1,0 mm.
